# EUROPEAN PATENT SPECIFICATION

(11) **EP 0 759 306 B1**
(45) Date of publication and mention of the grant of the patent: **18.09.2002**
(21) Application number: 96112902.0
(22) Date of filing: 09.08.1996
(51) Int. Cl.: A61M 15/00

(54) **Medicine administering apparatus**
Vorrichtung zum Verabreichen von Medikamenten
Dispositif d'administration de médicament

(30) Priority: 11.08.1995 JP 22726695
(43) Date of publication of application: 26.02.1997
(60) Divisional application: 01121188.5
(73) Proprietor: UNISIA JECS CORPORATION, Atsugi-shi, Kanagawa-ken 243-8510 (JP); DOTT LIMITED COMPANY, Yokohama-shi, Kanagawa 224 (JP)
(72) Inventor: Nakamura, Shigemi, Atsugi-shi, Kanagawa 243 (JP); Ishizeki, Kazunori, Atsugi-shi, Kanagawa 243 (JP); Ohki, Hisatomo, Atsugi-shi, Kanagawa 243 (JP); Yanagawa, Akira, Yokohama-shi, Kanagawa 224 (JP)
(74) Representative: Schoppe, Fritz, Dipl.-Ing.

(56) References cited:
- EP-A- 0 640 354
- GB-A- 2 165 159
- US-A- 2 587 215
- US-A- 5 239 993

## Description

This invention relates to improvements in a medicine administering apparatus suitable for administering, for example, powder-like and granular medicine to a patient.

In general, administering medicine into the lungs of an asthma patient or the like is carried out, for example, in a method to inject the medicine to the patient, in a method that the patient inhales the medicine by using a liquid aerosol sprayer, or in a method that the patient inhales fine and granular medicine (having a grain size, for example, ranging from 5 to 10 µm) filled in a capsule upon breaking the capsule. Of these medicine administration methods, the method that the patient inhales fine and granular medicine includes a manner to inhale medicine filled in a capsule upon breaking the capsule, and another manner that medicine in an amount corresponding to a plurality of doses is accommodated in a medicine administering apparatus, upon which the medicine in an amount corresponding to one dose is measured and taken out to be administered to the patient.

The latter (another) manner of administering the medicine to the patient is, for example, employed in a medicine administering apparatus disclosed in Japanese Patent Publication (Tokuhyohei) 3-504457. This medicine administering apparatus includes a disc-shaped administering unit formed with a plurality of administering holes. The administering unit is located at the bottom section of a material accommodating container in which power material such as medicine or the like is accommodated, so that each administering hole of the administering unit is charged with the material. A scraper is disposed in contact with the upper surface of the administering unit, in which the scraper scrapes an excessive portion of the material projected above each administering hole by rotating the administering unit.

In order to carry out medicine administration using this medicine administering apparatus, first the medicine as the power material is accommodated within the material accommodating container, and then the medicine is dropped into each administering hole of the administering unit. Thereafter, the excess amount of the medicine projected above each administering hole is scraped by the scraper thereby measuring and distributing the medicine in an amount corresponding to one dose. In this state, a patient holds the mouth piece of the medicine administering apparatus in the mouth and inhales the distributed medicine through the mouth piece. A medicine administering apparatus as disclosed in Japanese Patent Publication No. 1-47190 is constructed and arranged similarly to that discussed above and disclosed in Japanese Patent Publication No. 3-504457.

Additionally, the above medicine administration manner that the medicine in an amount corresponding to one dose is measured and taken out from the medicine in an amount corresponding to the plural doses is used also in a medicine administering apparatus disclosed in Japanese Patent Provisional Publication No. 59-88158. This medicine administering apparatus includes a blister case having a plurality of blisters which are circularly arranged. Each blister contains medicine corresponding to one dose and adapted to be opened when each dose is to be made, so that the medicine in the blister is inhaled by a patient.

Now, the above-discussed conventional medicine administering apparatuses are arranged such that medicine is dropped into the administering hole or the like under gravity thereby distributing the medicine in the amount corresponding to one dose. Accordingly, each medicine administering apparatus is required to take a predetermined vertical locational relationship in use thereof, so that the medicine cannot be accurately distributed under an inclined state of the medicine administering apparatus. This requires the patient to raise the body when medicine administration is made, thereby rendering a medicine administration operation troublesome.

Of the above conventional medicine administering apparatuses, ones disclosed in Japanese Patent Publication No. 3-504457 and Japanese Patent Publication No. 1-47190 is arranged such that the medicine is dropped into each medicine administering hole and followed by scraping the excess medicine. However, there is the possibility that granular medicine partly cakes owing to humidity or the like, so that a difference in density is made between a caked part and a granular part in the medicine. As a result, even though the medicine is distributed for each dose in the same way, difference is unavoidably made in actual amount of the medicine among respective portions distributed to be dosed. This makes it impossible to administer a predetermined amount of the medicine to the patient.

Difficulties have been encountered also in the above-discussed conventional medicine administering apparatus disclosed in Japanese Patent Provisional Publication No. 59-88158, in which the blister case has a plurality of the blisters for a plurality of doses. That is, the number of the blisters formed in the blister case is limited, and therefore a spare blister case is required to be carried by a patient. EP-A-0 640 354 discloses a whirl chamber powder inhaler forming a device for inhaling an aerosol. This device comprises a reservoir for storing the supply of medium for inhaling, an inhaling piece for sucking air along a dose of medium for inhaling and transporting means for transporting the dose of medium for inhaling out of the reservoir, wherein a whirl chamber is incorporated in the inhaling piece for taking the medium into the air with only a small suction force.

US-A-5,239,993 discloses an inhalator having a medicine storage chamber provided in a housing. In the chamber an isolation plate and a biasing spring are provided for compressing powder compound held in the medicine storage chamber. A fixed amount of medicine is transferred into a transfer chamber, and by a lateral movement of a mechanism the medicine is transferred into a swirl chamber.

It is the object underlying the present invention to provide an improved medicine administering apparatus which effectively overcomes drawbacks encountered in conventional medicine administering apparatus which avoids any directional restrictions with respect to the use of such an apparatus and provides for an apparatus enabling a provision of the medicine always in a constant density and with a predetermined amount.

This object is achieved by a medicine-administering apparatus according to claim 1.

According to one advantage, the present invention provides an improved medicine administering apparatus which is largely facilitated in its handling or operability while being improved in medicine administering efficiency.

According to a further advantage the present invention provides an improved medicine administering apparatus which has no directional restriction in use while making it possible to securely distribute a predetermined amount of medicine to be administered at each dose.

A medicine administering apparatus of the present invention comprises a main body which has a medicine charging chamber, and a medicine passage. The location of the medicine charging chamber is changed over between a first position where the medicine charging chamber is separate from the medicine passage and a second position where the medicine charging chamber is in the medicine passage. A predetermined amount of medicine is charged in a compressed state into the medicine charging chamber at the first position, the predetermined amount corresponding to one dose. The medicine within the medicine charging chamber is supplied through the medicine passage to a patient.

With this arrangement, the medicine in an amount corresponding to one dose is charged in the compressed state into the medicine charging chamber of the main body of the medicine administering apparatus, and therefore the charged medicine is caked in the medicine charging chamber so that the medicine in the medicine charging chamber can always take a constant density. Accordingly, the medicine can be charged into the medicine charging hole regardless of inclination or directional relationship of the medicine administering apparatus, thereby omitting a directional restriction of the medicine administering apparatus in use. Thus, the medicine administering apparatus can be largely improved in operability. Additionally, by changing over the location of the medicine charging chamber into the medicine passage, the medicine in an amount corresponding to one dose can be administered to the patient thereby dosing a predetermined amount of the medicine to the patient, thus improving the effects of the medicine. As discussed above, the medicine is charged in its caked state in the medicine charging hole, and therefore the medicine can be easily administered to the patient who is left lying in a bed without raising the body during medicine administration.

### BRIEF DESCRIPTION OF THE DRAWINGS

In the drawings, same reference numerals designate same parts and elements throughout all figures:
Fig. 1 is a vertical sectional view of a first embodiment of a medicine administering apparatus according to the present invention;
Fig. 2 is a sectional view taken in the direction of arrows substantially along the line II-II of Fig. 1;
Fig. 3 is an enlarged left-side view of a passage member in the medicine administering apparatus of Fig. 1;
Fig. 4 is a fragmentary enlarged vertical sectional view of an essential part of the medicine administering apparatus of Fig. 1, showing an operational state in which medicine is forcibly charged into a medicine charging hole at a medicine charging position under the action of a medicine stuffing mechanism;
Fig. 5 is a fragmentary enlarged vertical sectional view similar to Fig. 4 but showing another operational state in which the medicine charging hole in the state of Fig. 4 is changed into a medicine administering position;
Fig. 6 is a vertical sectional view similar to Fig. 1 but showing an operational state of the medicine administering apparatus of Fig. 1, in which the medicine in the medicine charging hole at the medicine administering position of Fig. 5 is inhaled; and
Fig. 7 is a fragmentary enlarged vertical sectional view of an essential part of a second embodiment of the medicine administering apparatus according to the present invention.

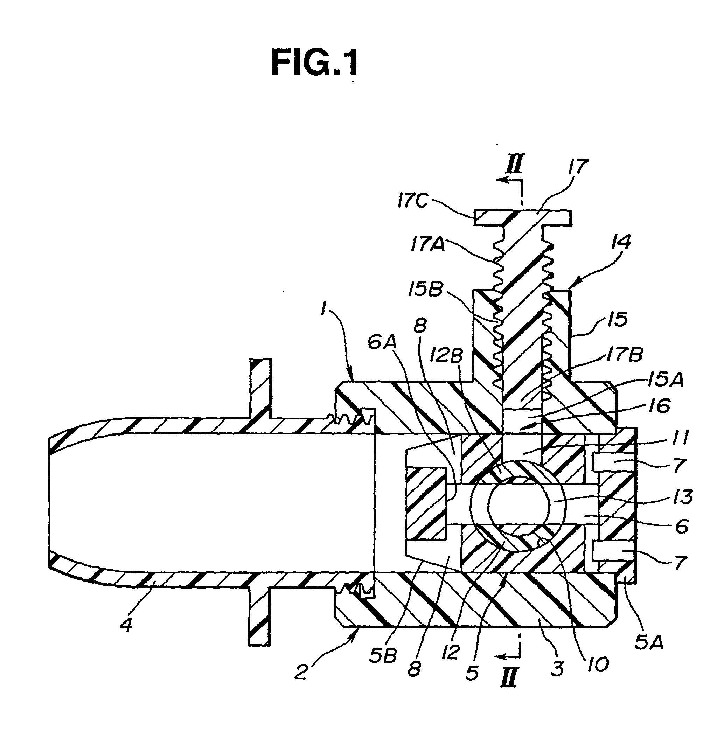

Referring now to Figs. 1 to 6, more specifically to Figs. 1 to 3, of the drawings, a first embodiment of a medicine administering apparatus according to the present invention is illustrated by the reference numeral 1. The medicine administering apparatus 1 comprises a main body 2 which includes a generally cylindrical inhaling body 3 in which a passage member 5 is fitted. A mouth piece 4 is detachably threadedly connected to inhaling body 3. A hopper 15 (discussed after) is formed integral with the inhaling body 3 to project radially outwardly from the outer peripheral surface of the inhaling body 3. The inhaling body 3 is formed with a fitting hole 3A which is located at a position separate 90 degrees from the hopper 15 in a peripheral direction of the inhaling body 3. The fitting hole 3A is formed piercing the cylindrical wall of the inhaling body 3 so as to be opened at the outer peripheral surface of the inhaling body 3. A change-over cylindrical member 12 is rotatably supported within the fitting hole 3A.

The passage member 5 is disposed within the inhaling body 3 and formed generally column-shaped. The passage member 5 is integrally formed at the outer periphery of its one end with an annular stopper section 5A which functions to locate the passage member 5 in the suction body 3 upon being brought into contact with one end of the inhaling body 3. The other end of the passage member 5 is formed generally frustoconical so as to have a tapered surface 5B whose diameter gradually decreases in a direction away from the annular stopper section 5A.

A medicine passage 6 is formed coaxial with the passage member 5 and extends along the center axis of the passage member 5. As shown in Fig. 1, the medicine passage 6 extends from the wall of the one end portion of the passage member 5 to the wall of the other end portion of the passage member 5. The one end portion of the passage member 5 is formed with two air inflow-side passages 7, 7 which are located at the diametrically opposite sides of and communicated with the medicine passage 6. The other end portion of the passage member 5 is formed with two outflow-side passages 8 which are opened to the tapered surface 5B. The outflow-side passages 8 are formed by cutting out the frustoconical end portion of the passage member 5 through the tapered surface 5B. The outflow-side passages 8 are located at the diametrically opposite sides of and communicated with the medicine passage 6.

Thus, the inflow-side passage 7 are formed such that air is flown into the medicine passage 6 from the diametrically opposite outsides to generate two opposite air streams which strike against each other in the medicine passage 6 thereby producing a turbulence flow of air in the medicine passage 6. As a result, granular medicine in the medicine passage 6 can be effectively dispersed. Additionally, the wall of the other end portion of the passage member 5 serves as a medicine striking surface 6A to which the dispersed medicine strikes upon being carried by air stream flowing through the medicine passage 6, so that the medicine can be further finely pulverized.

The passage member 5 is further formed with two auxiliary air flow passages 9, 9 which are formed at the diametrically opposite sides of the medicine passage 6. Each auxiliary air flow passage 9 is located at a position separate 90 degrees in angle from each inflow-side passage 7 or outflow-side passage 8 in a peripheral direction of the passage member 5. Each auxiliary air flow passage 9 axially pieces the passage member 5 so as to extend from the tip end surface of the one end portion to the tip end surface of the other end portion of the passage member 5 in order to increase the amount of air to be sucked to a patient or user when the patient breathes in through the medicine administering apparatus 1 thereby avoiding occurrence of difficulty in breathing.

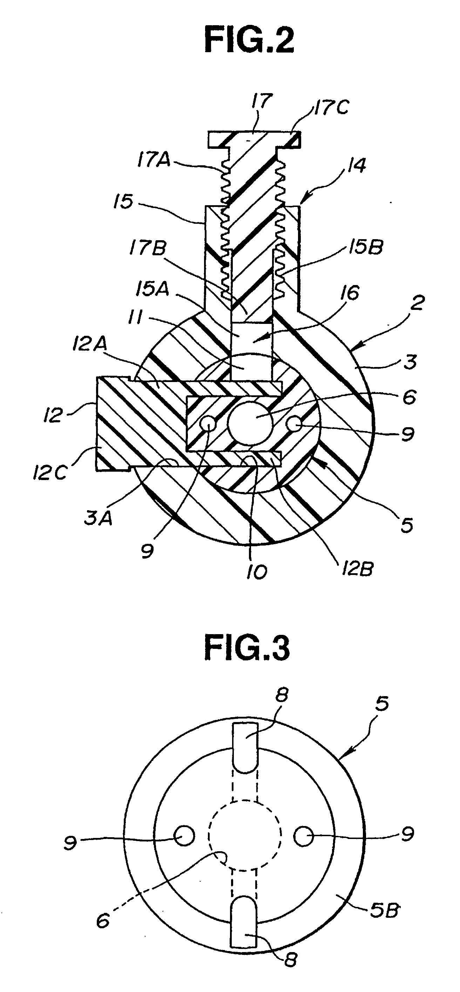

An annular groove 10 is formed in the passage member 5 which diametrically extends so that one end thereof is opened to the outer peripheral surface of the passage member 5 while the other end thereof is positioned in the solid part of the passage member 5 so as not to reach the outer peripheral surface of the passage member 5. The annular groove 10 extends across the medicine passage 6 and one (at the left side in Fig. 2) of the auxiliary air flow passage 9, and located in such a manner that the axis of the annular groove 10 is perpendicular to that of the medicine passage 5. The annular groove 10 is coaxial with a cylindrical insertion hole 3A formed through the cylindrical wall of the inhaling body 3. The insertion hole 3A is coaxial with the annular groove 10 and formed in axial coincidence with each other.

A medicine accommodating hole 11 is formed in the passage member 5 to radially extend so that one end thereof is opened to the outer peripheral surface of the passage member 5 while the other end thereof is opened to the annular groove 10. The medicine accommodating hole 11 is located such that the extension of the axis thereof is perpendicular to the axis of the medicine passage 6. The medicine accommodating hole 11 has the same diameter as the medicine passage 6. The medicine accommodating hole 11 is further coincident with a medicine accommodating hole 15A formed in the inhaling body 3 to constitute a medicine accommodating chamber 16, in which the medicine accommodating hole 15A has the same diameter as the medicine accommodating hole 11. The medicine in an amount corresponding to a plurality of doses is accommodated in the medicine accommodating chamber 16.

The change-over cylindrical member 12 is disposed throughout the insertion hole 3A of the inhaling body 3 and the annular groove 10 of the passage member 5. The change-over cylindrical member 12 includes a cylindrical solid section 12A disposed within the insertion hole 3A of the inhaling body 3, and a cylindrical section 12B which coaxially extends from the solid section 12A and is rotatably fitted in the annular groove 10 maintaining a granule or powder tight seal. The solid section 12A has a dial portion 12C which is projected out of the insertion hole 3A of the inhaling body 3, so that the change-over cylindrical member 12 is rotationally operated upon being rotated through the dial portion 12C by the patient or user.

Two medicine charging holes (chambers) 13, 13 are formed through the cylindrical wall of the cylindrical section 12B of the change-over cylindrical member 12. The two medicine charging holes 13, 13 are located at the opposite sides of the axis of the cylindrical section 12B and be able to be brought into coincidence with the medicine passage 6 and with the medicine accommodating hole 11 by rotating the dial portion 12C of the change-over cylindrical member 12 by 90 degrees in angle. Thus, the rotational position of the medicine charging hole 13 can be changed over by rotating the dial portion 12C so as to change over the communication between the medicine charging hole 13 and the medicine accommodating hole 11 to the communication between the medicine charging hole 13 and the medicine passage 6 and vice versa. In other words, the medicine charging hole 13 can be selectively put into coincidence with the medicine accommodating hole 11 and the medicine passage 6. The medicine charging hole 13 has the same diameter as the medicine accommodating hole 11 and as the medicine passage 6. The volume of the medicine changing hole 13 defined between the inner and outer peripheral surfaces of the cylindrical section 12B of the change-over cylindrical member 12 is set to be charged with the medicine (in a compressed state) in a predetermined volume required for each dose.

A medicine stuffing mechanism 14 is provided in the medicine administering apparatus 1 to stuff or compressedly charge the medicine into the medicine accommodating chamber 16. The medicine stuffing mechanism 14 includes the hopper 15 which generally cylindrical and integral with the inhaling body 3. The hopper 15 projects radially outwardly to have a tip end separate from the inhaling body 3. The hopper 15 is formed at its most inner surface with an internal thread 17A. The inner peripheral surface defined by the internal thread 17A is aligned and communicated with the medicine accommodating hole 15A in the inhaling body 3.

A pusher 17 is formed generally column-like and includes a rod-like pushing section 17B which is formed at its outer peripheral surface with an external thread 17A engageable with the internal thread 15B of the hopper 15. A dial section 17C is formed at one end of the pushing section 17B which end is projected from the tip end of the hopper 15. The pushing section 17B is screwed in the axial hole of the hopper 15 by turning the dial section 17C, in which the medicine within the medicine accommodating chamber 16 is stuffed and compressed by the tip end of the pushing section 17B.

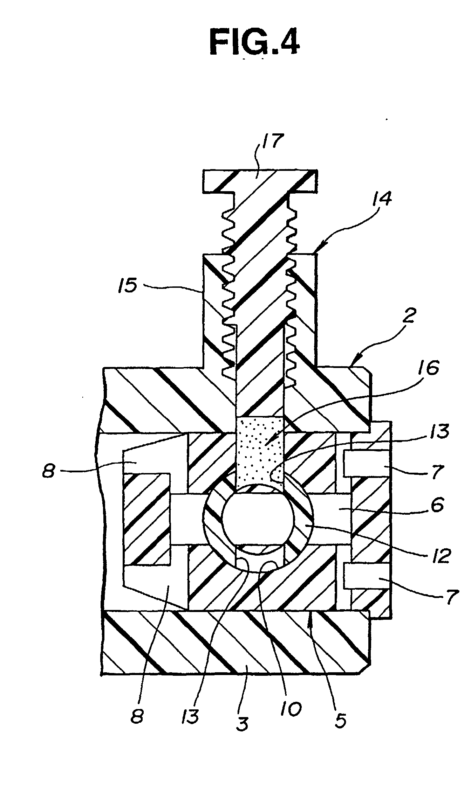

Next, the manner of operation of the above medicine administering apparatus 1 will be discussed with reference to particularly Figs. 4 to 6.

First, the change-over cylindrical member 12 is turned by turning its dial portion 12C so as to allow either one of the medicine charging holes 13 in the change-over cylindrical member 12 to come into coincidence (communication) with the medicine accommodating chamber 16, i.e., to come into a medicine charging position where the medicine is stuffed or charged into the medicine charging hole 13. In this state, the pusher 17 is turned by a predetermined rotational angle, so that the granular medicine in an amount corresponding to one dose is stuffed or charged in the medicine changing hole 13 in the change-over cylindrical member 12 as shown in Fig. 4. The medicine has been already stuffed in the medicine accommodating chamber 16 under the action of the pusher 17.

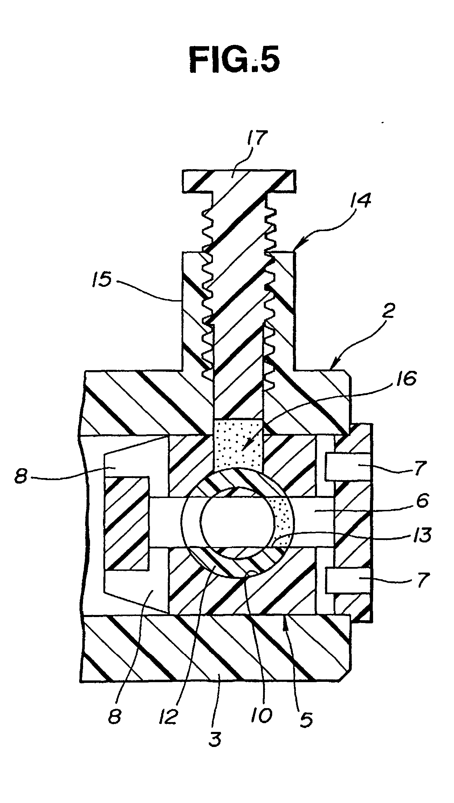

Upon completion of charging the medicine in the amount corresponding to one dose, the change-over cylindrical member 12 is turned right by a rotational angle of 90 degrees, so that the medicine changing hole 13 is located to come into coincidence with the medicine passage 6, i.e., to come into a medicine administering position where administration of the medicine is made, as shown in Fig. 5. At this time, the cylindrical section 12B of the change-over cylindrical member 12 is fitted in the annular groove 10 maintaining the power tight seal, and therefore the medicine overflown from the medicine charging hole 13 is scraped at the outer peripheral surface of the annular groove 10 when the medicine charging hole 13 is changed from the medicine charging position to the medicine administering position. As a result, the medicine in the amount corresponding securely to one dose can be measured and distributed to the side of the medicine passage 6.

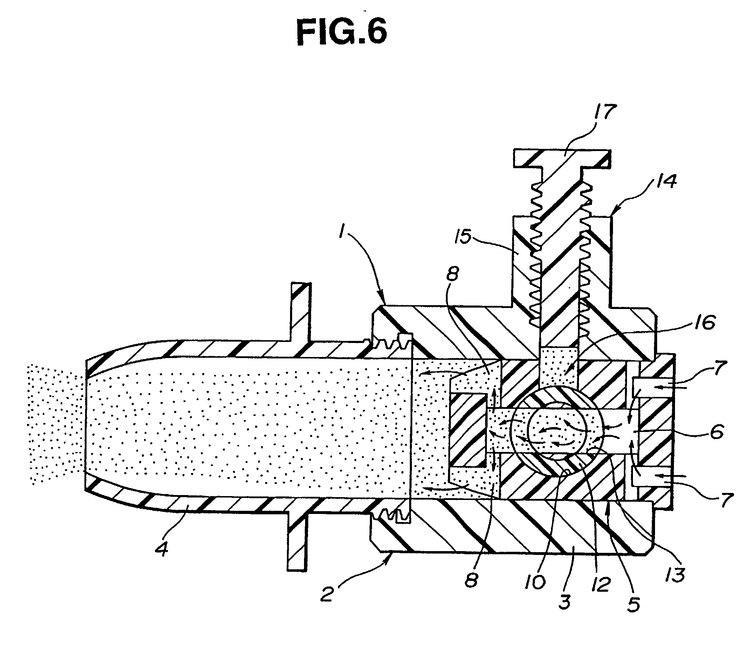

After the above preparation operation has been completed, the patient holds the mouth piece 4 in the mouth and breathes in, so that air flows through the inflow-side passages 7 into the medicine passage 6 as shown in Fig. 6. Then, the granular medicine filled in the medicine charging hole 13 of the cylindrical section 12B of the change-over cylindrical member 12 is dispersed to form an air stream mixing with the medicine. At this time, two air streams flow into the medicine passage 6 from opposite (radially inward) directions, and therefore the turbulence flow of air is generated within the medicine passage 6 thereby effectively dispersing the granular medicine. Additionally, the thus dispersed medicine flows axially and strikes against the medicine striking surface 6A, so that even a part of the medicine which has been left in an aggregate state can be securely finely pulverized to be effectively dispersed in air stream.

Air stream containing the medicine is released through the outflow-side passages 8 into the inside of the mouth piece 4 as shown in Fig. 6, and then reaches the inside of the lungs through the inside of the mouth and the trachea of the patient. Thus, the medicine in air stream can be administered to the lungs of the patient.

When the next medicine administration operation is to be made, the change-over cylindrical member 12 is turned 90 degrees in rotational angle to restore the medicine charging hole 13 to the medicine charging position, in which the above operations made after the medicine charging operation is to be repeated thereby repeatedly accomplishing administration of the medicine in the measured amount. Thus, a plurality of doses of the medicine are achieved.

Advantageous effects of the medicine administering apparatus of this embodiment will be discussed.

According to this embodiment, the medicine within the medicine accommodating chamber 16 is compressed to a predetermined density under the action of the pusher 17 and then charged into the medicine charging hole 13, and therefore the medicine can be charged into the medicine charging hole 13 for the purpose of distributing the medicine in the amount corresponding to one dose even when the medicine stuffing mechanism 14 is located at the lower side of the medicine charging hole 13. This makes it possible to operate the medicine administering apparatus 1 in any directions thereby greatly improving the operability of the medicine administering apparatus 1.

Additionally, since the medicine is charged in its compressed state into the medicine charging hole 13 so as to keep the medicine in a lumped state in the medicine charging hole 13, it is made possible to administer the medicine even in a condition where the medicine administering apparatus 1 is kept in a position where the axis of the main body 2 is vertical so that the mouth piece 4 is located below. This makes it possible to readily accomplish a medicine administration even under a condition where the patient lies in a bed without raising the body of the patient.

Further, the medicine in the amount corresponding to one dose is charged into the medicine charging hole 13 under a condition where the whole medicine has been staffed to obtain a predetermined density. As a result, the amount of the medicine for each dose can be prevented from being non-uniform due to difference in density, thereby facilitating to administer a predetermined amount of the medicine to the patient thus to raise the effects of the medicine.

Furthermore, the medicine overflown from the medicine charging hole 13 can be scraped at the outer peripheral surface of the annular groove 10 when the medicine charging hole 13 is changed from the medicine charging position to the medicine administering position. Consequently, the medicine in the amount which corresponds securely to one dose can be measured and distributed to the side of the medicine passage 6 in the passage member 5, thus improving an administering efficiency.

Besides, according to the medicine administering apparatus of this embodiment, the medicine charging hole 13 can be changed from the medicine charging position to the medicine administering position or vice versa under the turning action of the change-over cylindrical member 12. This makes the minimum a space necessary for accomplishing the change between the above two positions, thereby making the medicine administering apparatus small-sized.

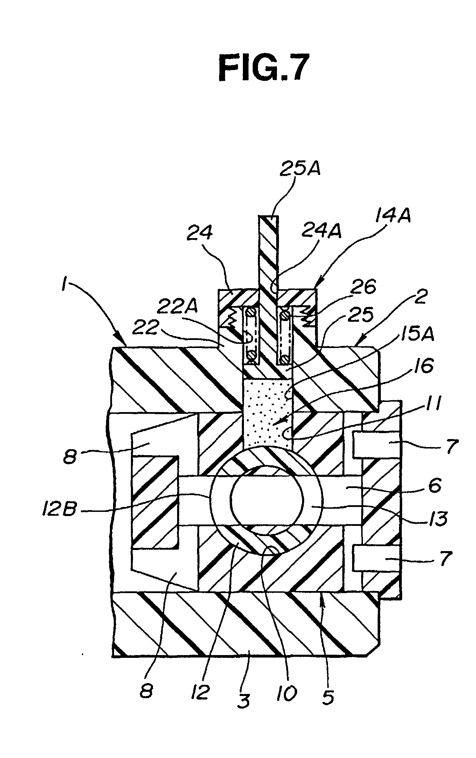

Fig. 7 illustrates a second embodiment of the medicine administering apparatus 1 according to the present invention, which is similar to the first embodiment medicine administering apparatus 1 with the exception that a medicine stuffing mechanism 14A is used in place of the medicine stuffing mechanism 14 of the first embodiment. The medicine stuffing mechanism 14A includes a generally cylindrical hopper 22 which is integral with the inhaling body 3 and projects radially outwardly from the outer peripheral surface of the inhaling body 3. The hopper 22 is formed with an axial central bore 22A which is aligned with and has the same diameter as the medicine accommodating chamber 16.

A cap 24 is threadedly attached to the tip end of the hopper 22 and formed with a central guide hole 24A. A piston 25 is movably disposed within the bore 22A of the hopper 22 and is insertable into the medicine accommodating chamber 16. The piston 25 is fixedly provided with a piston rod 25A which extends out of the cap 24 through the guide hole 24A. The piston 25 is movable in the axial direction of the medicine accommodating chamber 16 by moving the piston rod 25A. A spring member 26 is disposed within the axial bore 22A and located between the piston 25 and the cap 24. The biasing force of the spring member 26 is set at such a value as to obtain a predetermined density of the granular medicine within the medicine accommodating chamber 16.

With the above arrangement of this embodiment, first the medicine in an amount corresponding to a plurality of doses is supplied through the central bore 22A into the medicine accommodating chamber 16, and then the piston 25 is inserted into the central bore 22A by pushing the piston rod 25A. Thereafter, the spring member 26 and the cap 24 are successively assembled in position, in which the piston 25 is biased to push the medicine in the central bore 22A into the medicine accommodating chamber 16 so that the medicine is compressed to obtain a predetermined density. The thus compressed medicine is charged into the medicine charging hole 13 formed in the cylindrical section 12B of the change-over cylindrical member 12.

According to this embodiment, the generally same advantageous effects as the first embodiment can be attained. Besides, the medicine within the medicine accommodating chamber 23 is compressed under the biasing force of the spring member 26, and therefore the operations of compressing the medicine and charging the medicine into the medicine charging hole 13 can be automatically accomplished thereby improving the operability of the medicine administering apparatus 1.

## Claims

1. A medicine administering apparatus (1) comprising:
a main body (2) including means for defining a medicine charging chamber (13), and means for defining a medicine passage (6);
means (12) for changing over location of said medicine charging chamber (13) between a first position where said medicine charging chamber (13) is separate from said medicine passage (6) and a second position where said medicine charging chamber (13) is in said medicine passage (6);
means (15; 17) for charging a predetermined amount of medicine in a compressed state into said medicine charging chamber (13) at the first position, said predetermined amount corresponding to one dose;
means (4) for supplying the medicine within said medicine charging chamber (13) through said medicine passage (6) to a patient; and
wherein said changing over means (12) includes a cylindrical member (12) rotatably supported in said main body (2) and formed with a hole formed through a wall of said cylindrical member (12), said hole serves as said medicine charging chamber (13), location of said hole being changed between a first position where said hole is in communication with a medicine accommodating chamber (16) and a second position where said hole is in communication with said medicine passage (6).

2. A medicine administering apparatus (1) as claimed in Claim 1, further comprising means defining said medicine accommodating chamber (16) in which the medicine in an amount corresponding to a plurality of doses is accommodated, said medicine accommodating chamber (16) being in communication with said medicine charging chamber (13).

3. A medicine administering apparatus (1) as claimed in Claim 2, wherein said medicine charging means (15, 17) includes a rod-like pushing member (17B) formed with an external thread (17A) which is in engagement with an internal thread (17A) formed in said main body (2), a tip end of said pushing member (17) defining said medicine accommodating chamber (16), said pushing member (17) being axially moved to compress the medicine in said medicine accommodating chamber (16) upon being turned.

4. A medicine administering apparatus (1) as claimed in Claim 2, wherein said medicine charging means includes a piston (25) slidably disposed in a bore (22A) formed in said main body (3), said piston (25) defining a medicine accommodating chamber (16), and a spring (26) for biasing said piston (25) to compress the medicine within said medicine accomodating chamber (16).

## Patentansprüche

1. Eine Medizinverabreichungsvorrichtung (1), die folgende Merkmale aufweist:
einen Hauptkörper (2), der eine Einrichtung zum Definieren einer Medizinbeschickungskammer (13) und eine Einrichtung zum Definieren eines Medizindurchgangs (6) umfaßt;
eine Einrichtung (12) zum Wechseln einer Lage der Medizinbeschickungskammer (13) zwischen einer ersten Position, bei der die Medizinbeschickungskammer (13) von dem Medizindurchgang (6) getrennt ist, und einer zweiten Position, bei der sich die Medizinbeschickungskammer (13) in dem Medizindurchgang (6) befindet;
eine Einrichtung (15; 17) zum Einfüllen einer vorbestimmten Menge an Medizin in einem komprimierten Zustand in die Medizinbeschickungskammer (13) in der ersten Position, wobei die vorbestimmte Menge einer Dosis entspricht;
eine Einrichtung (4) zum Zuführen der Medizin in der Medizinbeschickungskammer (13) durch den Medizindurchgang (6) zu einem Patienten; und
wobei die Wechseleinrichtung (12) ein zylindrisches Bauglied (12) umfaßt, das in dem Hauptkörper (2) drehbar getragen wird und mit einem Loch gebildet ist, das durch eine Wand des zylindrischen Bauglieds (12) gebildet ist, wobei das Loch als die Medizinbeschikkungskammer (13) dient, wobei die Lage des Loches zwischen einer ersten Position, bei der das Loch mit einer Medizinaufnahmekammer (16) in Kommunikation steht, und einer zweiten Position, bei der das Loch mit dem Medizindurchgang (6) in Kommunikation steht, gewechselt wird.

2. Eine Medizinverabreichungsvorrichtung (1) gemäß Anspruch 1, die ferner eine Einrichtung aufweist, die die Medizinaufnahmekammer (16) definiert, in der die Medizin in einer Menge, die einer Mehrzahl von Dosen entspricht, aufgenommen ist, wobei die Medizinaufnahmekammer (16) in Kommunikation mit der Medizinbeschikkungskammer (13) steht.

3. Eine Medizinverabreichungsvorrichtung (1) gemäß Anspruch 2, bei der die Medizinbeschickungseinrichtung (15, 17) ein stabartiges Schiebebauglied (17B) umfaßt, das mit einem.Außengewinde (17A) gebildet ist, das mit einem Innengewinde (17A), das in dem Hauptkörper (2) gebildet ist, in Eingriff steht, wobei ein Spitzenende des Schiebebauglieds (17) die Medizinaufnahmekammer (16) definiert, wobei das Schiebebauglied (17) axial bewegt wird, um die Medizin in der Medizinaufnahmekammer (16), nachdem diese gedreht wurde, zu komprimieren.

4. Eine Medizinverabreichungsvorrichtung (1) gemäß Anspruch 2, bei der die Medizinbeschickungseinrichtung einen Kolben (25), der gleitbar in einer Bohrung (22A), die in dem Hauptkörper (3) gebildet ist, angeordnet ist, wobei der Kolben (25) eine Medizinaufnahmekammer (16) definiert, und eine Feder (26) zum Vorspannen des Kolbens (25), um die Medizin in der Medizinaufnahmekammer (16) zu komprimieren, umfaßt.

## Revendications

1. Dispositif d'administration de médicaments (1), comprenant:
un corps principal (2) comportant un moyen destiné à définir une chambre de chargement de médicament (13) et un moyen destiné à définir un passage de médicament (6);
un moyen (12) destiné à commuter l'emplacement de ladite chambre de chargement de médicament (13) entre une première position dans laquelle ladite chambre de chargement de médicament (13) est éloignée dudit passage de médicament (6) et une seconde position dans laquelle ladite chambre de chargement de médicament (13) se trouve dans ledit passage de médicament (6) ;
un moyen (15 ; 17) destiné à charger une quantité prédéterminée de médicament à l'état comprimé dans ladite chambre de chargement de médicament (13) dans la première position, ladite quantité prédéterminée correspondant à une dose ;
un moyen (4) destiné à alimenter le médicament à l'intérieur de ladite chambre de chargement de médicament (13), à travers ledit passage de médicament (6), vers un patient ; et
dans lequel ledit moyen de commutation (12) comporte un élément cylindrique (12) supporté de manière rotative dans ledit corps principal (2) et formé avec un trou formé dans une paroi dudit élément cylindrique (12), ledit trou sert de dite chambre de chargement de médicament (13), l'emplacement dudit trou étant changé entre une première position dans laquelle ledit trou se trouve en communication avec une chambre de logement de médicament (16) et une seconde position dans laquelle ledit trou se trouve en communication avec ledit passage de médicament (6).

2. Dispositif d'administration de médicaments (1) selon la revendication 1, comprenant, par ailleurs, un moyen destiné à définir ladite chambre de logement de médicament (16), dans lequel est logé le médicament en une quantité correspondant à une pluralité de doses, ladite chambre de logement de médicament (16) se trouvant en communication avec ladite chambre de chargement de médicament (13).

3. Dispositif d'administration de médicaments (1) selon la revendication 2, dans lequel ledit moyen de chargement de médicament (15 ; 17) comporte un élément poussoir en forme de tige (17B) formé avec un filetage (17A) qui vient en prise avec un taraudage (17A) formé dans ledit corps principal (2), une extrémité de pointe dudit élément poussoir (17) définissant ladite chambre de logement de médicament (16), ledit élément poussoir (17) étant déplacé axialement, pour comprimer le médicament dans ladite chambre de logement de médicament (16), après qu'il ait été tourné.

4. Dispositif d'administration de médicaments (1) selon la revendication 2, dans lequel ledit moyen de chargement de médicament comporte un piston (25) disposé de manière coulissable dans un alésage (22A) formé dans ledit corps principal (3), ledit piston (25) définissant une chambre de logement de médicament (16), et un ressort (26) destiné à dévier ledit piston (25), pour comprimer le médicament à l'intérieur de ladite chambre de logement de médicament (16).
